(19) Europäisches Patentamt
European Patent Office
Office européen des brevets

(11) **EP 4 704 090 A1**

(12) **EUROPEAN PATENT APPLICATION**
published in accordance with Art. 153(4) EPC

(43) Date of publication:
**04.03.2026  Bulletin 2026/10**

(21) Application number: **24796628.6**

(22) Date of filing: **15.03.2024**

(51) International Patent Classification (IPC):
**G16C 10/00** (2019.01)

(52) Cooperative Patent Classification (CPC):
**G16C 10/00**

(86) International application number:
**PCT/JP2024/010198**

(87) International publication number:
**WO 2024/224870 (31.10.2024 Gazette 2024/44)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **27.04.2023  JP 2023072880**

(71) Applicant: **Fujitsu Limited
Kawasaki-shi, Kanagawa 211-8588 (JP)**

(72) Inventor: **OHTA, Eiji
Kawasaki-shi, Kanagawa 211-8588 (JP)**

(74) Representative: **Haseltine Lake Kempner LLP
Cheapside House
138 Cheapside
London EC2V 6BJ (GB)**

(54) **QUANTUM CHEMICAL CALCULATION PROGRAM, QUANTUM CHEMICAL CALCULATION METHOD, AND INFORMATION PROCESSING DEVICE**

(57)    The computational complexity for determining a stable structure of a substance is reduced.

An information processing apparatus 10 initially sets the positions of a plurality of atoms constituting a substance in position information 2 indicating the positions of the plurality of atoms, and iteratively updates the positions of the plurality of atoms. When the positions of the plurality of atoms are initially set or updated, the information processing apparatus 10 calculates the total energy of the substance. When the positions of the plurality of atoms are initially set or updated, the information processing apparatus 10 calculates the magnitude of the force acting on each of the plurality of atoms. When the positions of the plurality of atoms are updated, the information processing apparatus 10 calculates an energy change rate indicating the degree of a change in the total energy of the substance before and after the updating. Then, when at least one of a first convergence condition 3 related to the magnitude of the force and a second convergence condition 4 related to the energy change rate is satisfied, the information processing apparatus 10 terminates the updating of the positions of the plurality of atoms and acquires the position information 2 at the termination.

FIG. 1

EP 4 704 090 A1

## Description

Technical Field

The embodiments discussed herein relate to a quantum chemical computation program, a quantum chemical computation method, and an information processing apparatus.

Background Art

In recent years, materials informatics (hereinafter referred to as "MI") has been advancing worldwide in the field of materials development as a data-driven research and development approach aimed at shortening research and development periods and reducing costs. In MI, the accumulation of high-quality material data is important. Therefore, in addition to experimental data, efficient data accumulation through simulations such as quantum chemical computation is expected.

One of the major computation methods in quantum chemistry is density functional theory (DFT). In DFT, the electron density of a target substance is first calculated using a self-consistent field (SCF) method. Then, based on the obtained electron density, physical quantities indicating the state of the substance (such as total energy) are calculated.

In materials development, DFT is used for structure optimization to determine a stable structure of a molecule that governs the properties of a substance. Structure optimization involves iteratively performing DFT while gradually changing the three-dimensional structure of a molecule, to search for a stable structure in which the total energy is minimized.

The computational complexity of DFT is $O(N^3)$ in order notation, where N denotes the number of atoms (N is a natural number). The computational complexity becomes enormous as the size of a target system increases. Therefore, to determine a stable structure of a molecule using DFT, it is desirable to reduce the computational complexity as much as possible.

As a technique for determining a stable structure of a substance, for example, a molecular design support system has been proposed that parallelizes the calculation of atomic coordinate derivatives of the total energy to determine a stable structure of a molecule at high speed and with low memory usage.

Citation List

Patent Literature

PTL1: Japanese Laid-open Patent Publication No. 06-187404

Summary of Invention

Technical Problem

In a calculation of structure optimization for determining a stable structure of a substance, it may take a long time until a convergence condition in the calculation is satisfied. For example, in structure optimization, for one or more atoms arranged in space or in a coordinate system, correction of the atomic arrangement and calculation of total energy by DFT for the atomic arrangement are iteratively performed until the force (strain) acting on all atoms converges. In the calculation process of such structure optimization, there is a case where the total energy transitions to minute fluctuations. When the total energy transitions to minute fluctuations, it becomes difficult for the force on some atoms to converge. As a result, the number of repetitions of the DFT calculation increases, and the computation time becomes prolonged.

In one aspect, the present disclosure aims to reduce the computational complexity for determining a stable structure of a substance.

Solution to Problem

According to one aspect, there is provided a quantum chemical computation program that causes a computer to perform the following process.

The computer initially sets positions of a plurality of atoms constituting a substance in position information indicating the positions of the plurality of atoms, and iteratively updates the positions of the plurality of atoms in a direction in which a state of the substance becomes stabilized. In response to the positions of the plurality of atoms being initially set or updated, the computer calculates a total energy of the substance based on the position information. In response to the positions of the plurality of atoms being initially set or updated, the computer calculates a magnitude of a force acting on each of the plurality of atoms based on the position information. In response to the positions of the plurality of atoms being updated, the computer calculates an energy change rate indicating a degree of a change in the total energy of the substance before and after the updating. The computer determines whether at least one of a first convergence condition related to the magnitude of the force and a second convergence condition related to the energy change rate is satisfied. Upon determining that at least one of the first convergence condition and the second convergence condition is satisfied, the computer terminates the updating of the positions of the plurality of atoms, and acquires the position information at termination.

Advantageous Effects of Invention

According to one aspect, the computational complexity for determining a stable structure of a substance is reduced.

Brief Description of Drawings

[FIG. 1] FIG. 1 illustrates an example of a quantum chemical computation method according to a first embodiment.
[FIG. 2] FIG. 2 illustrates an example of a system configuration.
[FIG. 3] FIG. 3 illustrates an example of hardware of a control node.
[FIG. 4] FIG. 4 illustrates an example of a hardware configuration of a computing node.
[FIG. 5] FIG. 5 illustrates the relationship between the magnitude of energy of an atom and the ease of convergence of force.
[FIG. 6] FIG. 6 illustrates an example of the number of DFT iterations needed until a convergence condition of force is satisfied.
[FIG. 7] FIG. 7 illustrates an example of convergence determination in structure optimization using a logical OR of two convergence conditions.
[FIG. 8] FIG. 8 illustrates an example of the number of DFT iterations needed until two convergence conditions are satisfied.
[FIG. 9] FIG. 9 is a block diagram illustrating an example of functions implemented by a computing system.
[FIG. 10] FIG. 10 is a flowchart illustrating an example procedure for a structure optimization process.
[FIG. 11] FIG. 11 is a flowchart illustrating an example procedure for a structure optimization process.
[FIG. 12] FIG. 12 illustrates an example of speeding up a structure optimization calculation by additionally using a convergence condition related to an energy change rate.
[FIG. 13] FIG. 13 illustrates an example of a wide-area search technology service.

Description of Embodiments

Hereinafter, embodiments will be described with reference to the drawings. A plurality of embodiments may be combined unless they exclude each other.

[First Embodiment]

A first embodiment relates to a quantum chemical computation method that reduces the computational complexity for determining a stable structure (ground state) of a substance.

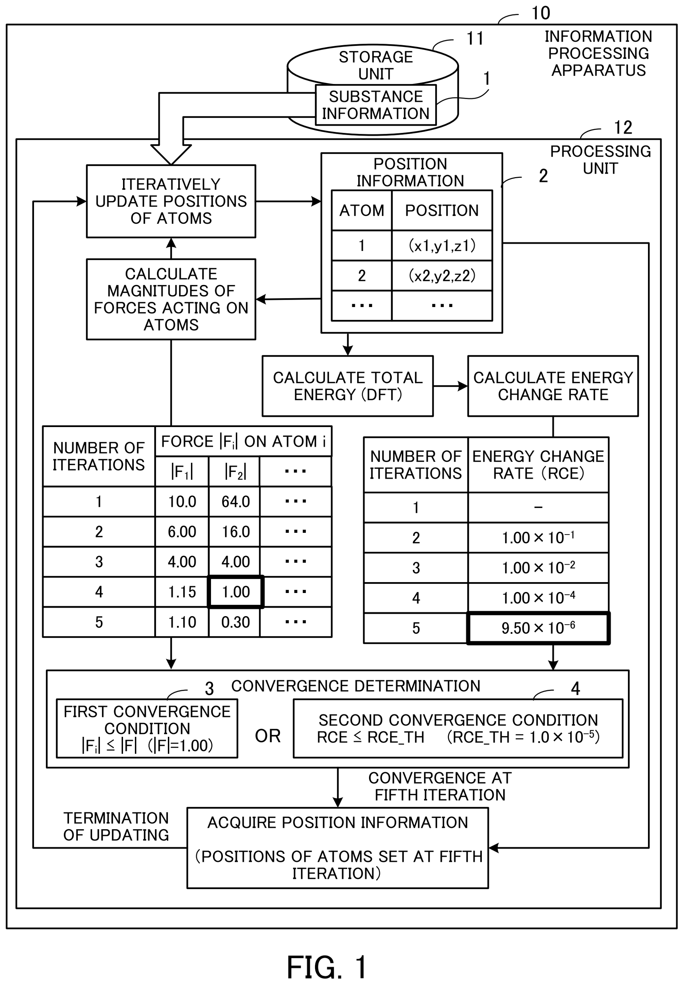

FIG. 1 illustrates an example of a quantum chemical computation method according to the first embodiment. FIG. 1 illustrates an information processing apparatus 10 that executes a quantum chemical computation method according to the first embodiment. The information processing apparatus 10 is able to implement the quantum chemical computation method according to the first embodiment by executing, for example, a quantum chemical computation program.

The information processing apparatus 10 includes a storage unit 11 and a processing unit 12. The storage unit 11 is, for example, a memory or a storage device included in the information processing apparatus 10. The processing unit 12 is, for example, a processor or an arithmetic circuit included in the information processing apparatus 10.

The storage unit 11 stores, for example, substance information 1 indicating a substance, for which a stable structure is to be determined. The substance information 1 indicates, for example, a molecular structure of the substance.

The processing unit 12 calculates the arrangement of atoms in a stable structure of the substance indicated by the substance information 1. For example, a substance has a stable structure, for example, when the substance has a minimum energy. A process of determining such a stable structure of a substance is called structure optimization. For example, the processing unit 12 performs the following process.

With regard to a substance specified by the substance information, the processing unit 12 initially sets the positions of a plurality of atoms constituting the substance in position information 2 indicating the positions of the plurality of atoms. Then, the processing unit 12 iteratively updates the positions of the plurality of atoms in a direction in which the state of the substance becomes stabilized.

When the positions of the plurality of atoms are initially set or updated, the processing unit 12 calculates the total energy of the substance based on the position information 2. For example, the processing unit 12 calculates the electron density in the space where the substance is present, by DFT and calculates the total energy based on the electron density.

When the positions of the plurality of atoms are initially set or updated, the processing unit 12 also calculates the magnitude of a force acting on each of the plurality of atoms based on the position information 2. For example, the processing unit 12 calculates, for each of the plurality of atoms, the sum of the potentials of the Coulomb force between that atom and each of the other atoms, based on the respective distances between the atom and the other atoms. The processing unit 12 calculates the magnitude of the force acting on each of the plurality of atoms, based on the sum of the potentials of that atom.

When the positions of the plurality of atoms are updated, the processing unit 12 calculates an energy change rate, also referred to as RCE, indicating the degree of a change in the total energy of the substance before and after the updating. For example, the processing unit 12 calculates the difference between the first total energy of the substance obtained based on the position information after the n-th updating (where n is a natural number) and a representative value of the second total energy of the substance obtained based on the position information after the m-th updating (where m is a natural number of n - 1 or less) to the (n-1)-th updating. Then, the processing unit 12 divides the calculated difference by the representative value of the second total energy, and sets the absolute value of the division result as the energy change rate.

When the magnitude of the force acting on each of the plurality of atoms and the energy change rate of the substance are obtained, the processing unit 12 then determines whether at least one of a first convergence condition 3 related to the magnitude of the force and a second convergence condition 4 related to the energy change rate is satisfied. This means that the convergence condition in the iterative process is the logical OR of the first convergence condition 3 and the second convergence condition 4.

For example, the processing unit 12 determines that the first convergence condition 3 is satisfied when the magnitude of the force is less than or equal to a first allowable value for all of the plurality of atoms. The processing unit 12 determines that the second convergence condition 4 is satisfied when the energy change rate is less than or equal to a second allowable value.

Then, if at least one of the first convergence condition 3 and the second convergence condition 4 is satisfied, the processing unit 12 terminates the process of updating the positions of the plurality of atoms, and acquires the position information 2 at the termination. The positions of the plurality of atoms set in the position information 2 acquired at this time indicate a stable structure of the substance.

As described above, when at least one of the first convergence condition 3 and the second convergence condition 4 is satisfied, the updating of the positions of the atoms is terminated, and the position information 2 at that time is acquired, whereby the computational complexity for determining the stable structure of the substance is reduced. That is, even if the magnitudes of forces acting on some of the atoms exceeds the first allowable value, if the energy change rate is less than or equal to the second allowable value, the iterative process of updating the positions of the atoms, calculating the total energy, calculating the magnitudes of forces, and calculating the energy change rate is terminated. Therefore, compared to the case where convergence is determined based only on the first convergence condition 3 related to the magnitudes of the forces acting on the atoms, for example, the convergence may be determined early, and the computational complexity is reduced.

For example, in the example of FIG. 1, the first allowable value "|F|" is "1.00", and the second allowable value "RCE_TH" is "$1.0 \times 10^{-5}$". At this time, the magnitude $|F_2|$ of the force acting on the second atom of the substance is less than or equal to the first allowable value at the fourth iteration. On the other hand, the magnitude $|F_1|$ of the force acting on the first atom of the substance exceeds the first allowable value even after the fifth iteration. Therefore, in the case of using the first convergence condition 3 only, the iterative process is not terminated even if the number of iterations reaches five.

The energy change rate is less than or equal to the second allowable value at the fifth iteration. Therefore, in the case of using the second convergence condition 4 as well in the convergence determination, the iterative process is terminated at the fifth iteration. As a result, the computational complexity until the stable structure of the substance is determined is reduced.

Examples of a case where it takes time for the force acting on any of the atoms to satisfy the first convergence condition 3 include a case where the energy of some of the atoms is low. In the case where the energy of an atom is low, the magnitude of the force acting on the atom repeatedly increases and decreases every time the position of the atom is updated, and it may take time to satisfy the first convergence condition 3.

During the repeated increases and decreases in the magnitude of the force acting on an atom, the amount of movement of the atom in the position updating of the atom increases when the magnitude of the force increases. However, even if the amount of movement of an atom with relatively low energy is somewhat large, the influence on the total energy is small. That is, even in a situation where the magnitude of the force acting on an atom with relatively low energy exceeds the first allowable value, if the energy change rate is sufficiently small to be less than or equal to the second allowable value, the influence of the increase or decrease in the magnitude of the force acting on the atom on the total energy is negligible. In such a case, it may be determined that the total energy is substantially at a minimum.

The positions of the atoms at which the total energy is at a minimum indicate a stable structure of the substance. That is, by performing the convergence determination in the iterative process based on the logical OR of the first convergence condition 3 and the second convergence condition 4, the processing unit 12 is able to reduce the computational complexity without reducing the accuracy of the finally determined stable structure of the substance.

In addition, in the case where the energy change rate used in the second convergence condition 4 is less than or equal to the second allowable value, it is ensured that the value of the total energy is sufficiently reduced. Therefore, by using the second convergence condition 4 related to the energy change rate, a user is able to appropriately evaluate the usefulness of the substance having the structure obtained through the structure optimization, based on the total energy of the substance.

[Second Embodiment]

A second embodiment relates to a computing system in which the number of DFT iterations is reduced in the case where a molecular structure of a substance is determined by DFT calculation using high-performance computing (HPC).

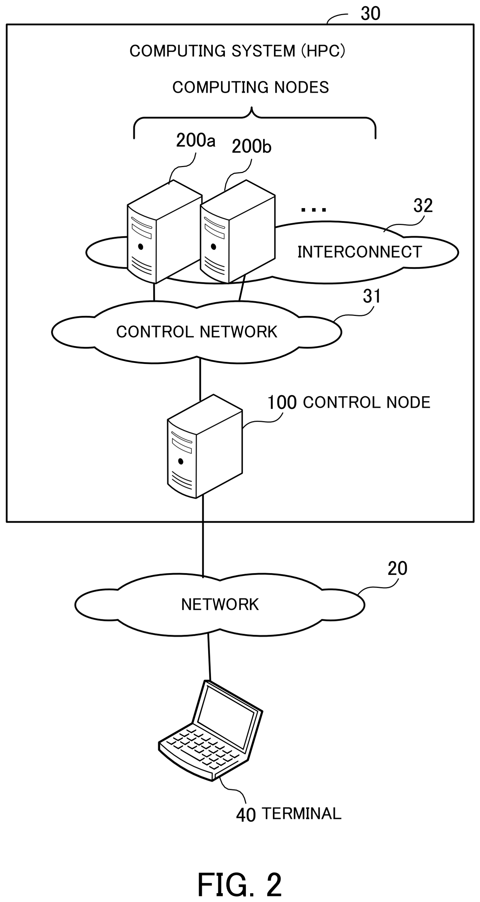

FIG. 2 illustrates an example of a system configuration. A computing system 30 that implements HPC includes a control node 100 and a plurality of computing nodes 200a, 200b, .... The plurality of computing nodes 200a, 200b, ... are connected with an interconnect 32 that enables the computing nodes 200a, 200b, ... to perform high-speed communication with each other. The interconnect 32 is, for example, a network in which processors are interconnected via a six-dimensional mesh/torus.

Each of the computing nodes 200a, 200b, ... is also connected to a control network 31. The control node 100 is further connected to the control network 31. The control node 100 is a computer that instructs the computing nodes 200a, 200b, ... to execute jobs.

The control node 100 is connected to a terminal 40 via a network 20. The terminal 40 is a computer that registers information on jobs to be executed by the computing nodes 200a, 200b, ... in the control node 100, based on user operations. The control node 100 instructs the plurality of computing nodes 200a, 200b, ... to perform DFT calculation in accordance with instructions from the terminal 40.

For example, the user is able to obtain a physical quantity such as the total energy of an electronic system defined by DFT, by causing the computing nodes 200a, 200b, ... to compute the state of a substance using DFT.

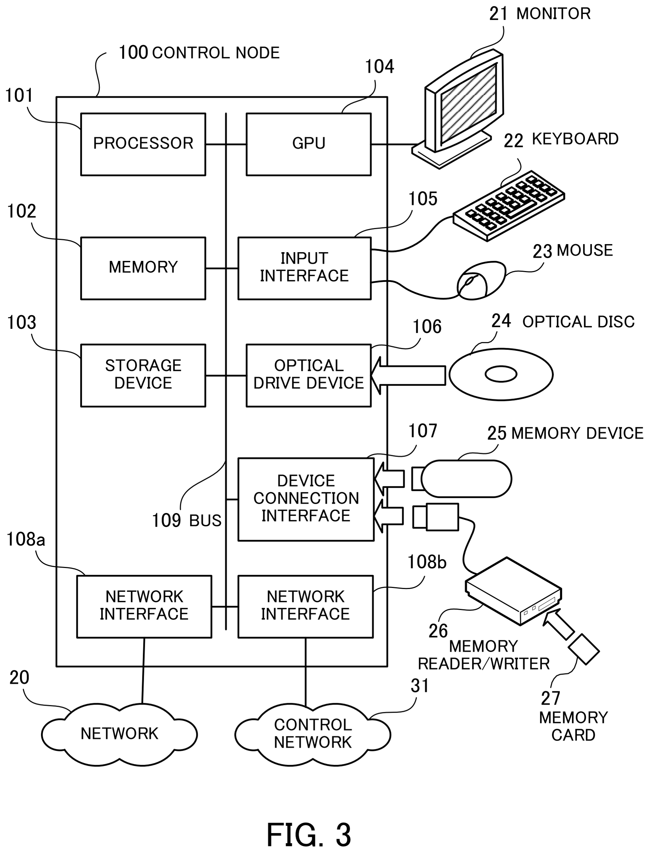

FIG. 3 illustrates an example of hardware of the control node. The control node 100 is entirely controlled by a processor 101. A memory 102 and a plurality of peripheral devices are connected to the processor 101 via a bus 109. The processor 101 may be a multiprocessor. The processor 101 is, for example, a central processing unit (CPU), a micro processing unit (MPU), or a digital signal processor (DSP). At least a part of the functions implemented by the processor 101 executing a program may be implemented by an electronic circuit such as an application specific integrated circuit (ASIC) or a programmable logic device (PLD).

The memory 102 is used as a main memory device of the control node 100. The memory 102 temporarily stores at least a part of operating system (OS) programs and application programs to be executed by the processor 101. The memory 102 also stores various data to be used for processing by the processor 101. As the memory 102, for example, a volatile semiconductor memory device such as a random access memory (RAM) is used.

The peripheral devices connected to the bus 109 include a storage device 103, a graphics processing unit (GPU) 104, an input interface 105, an optical drive device 106, a device connection interface 107, and network interfaces 108a and 108b.

The storage device 103 electrically or magnetically writes and reads data to and from a built-in recording medium. The storage device 103 is used as an auxiliary storage device of the control node 100. The storage device 103 stores OS programs, application programs, and various data. As the storage device 103, for example, a hard disk drive (HDD) or a solid state drive (SSD) may be used.

The GPU 104 is an arithmetic device that performs image processing, and is also called a graphic controller. A monitor 21 is connected to the GPU 104. The GPU 104 displays images on the screen of the monitor 21 in accordance with instructions from the processor 101. Examples of the monitor 21 include a display device using organic electro luminescence (EL) and a liquid crystal display device.

A keyboard 22 and a mouse 23 are connected to the input interface 105. The input interface 105 transmits signals received from the keyboard 22 and the mouse 23 to the processor 101. The mouse 23 is an example of a pointing device, and other pointing devices may be used. Examples of other pointing devices include a touch panel, a tablet, a touch pad, and a track ball.

The optical drive device 106 reads data recorded on an optical disc 24 or writes data to the optical disc 24 using laser light or the like. The optical disc 24 is a portable recording medium on which data is recorded so as to be readable by reflection of light. The optical disc 24 may be a digital versatile disc (DVD), a DVD-RAM, a compact disc read only memory (CD-ROM), a CD-recordable (CD-R), CD-rewritable (CD-RW), or the like.

The device connection interface 107 is a communication interface for connecting peripheral devices to the control node 100. For example, a memory device 25 and a memory reader/writer 26 may be connected to the device connection interface 107. The memory device 25 is a recording medium having a function of communicating with the device connection interface 107. The memory reader/writer 26 is a device that writes data to a memory card 27 or reads data from the memory card 27. The memory card 27 is a card-type recording medium.

The network interface 108a is connected to the network 20. The network interface 108a transmits and receives data to and from other computers and communication devices such as the terminal 40 via the network 20. The network interface 108b is connected to the control network 31. The network interface 108b transmits and receives data to and from the computing nodes 200a, 200b, ... via the control network 31.

Each of the network interfaces 108a and 108b is a wired communication interfaces connected to a wired communication device such as a switch or a router by a cable. Each of the network interfaces 108a and 108b may be a wireless communication interface that is communicatively connected to a wireless communication device such as a base station or an access point by radio waves.

The control node 100 implements the processing functions of the second embodiment by executing a program recorded on a computer-readable recording medium, for example. The program describing the processing contents to be executed by the control node 100 may be recorded on various recording media. For example, a program to be executed by the control node 100 may be stored in the storage device 103. The processor 101 loads at least a part of the program from the storage device 103 into the memory 102 and executes the program. The program to be executed by the control node 100 may be recorded in a portable recording medium such as the optical disc 24, the memory device 25, or the memory card 27. The program stored in the portable recording medium becomes executable after being installed in the storage device 103 under the control of the processor 101, for example. Alternatively, the processor 101 may read the program directly from the portable recording medium and execute the program.

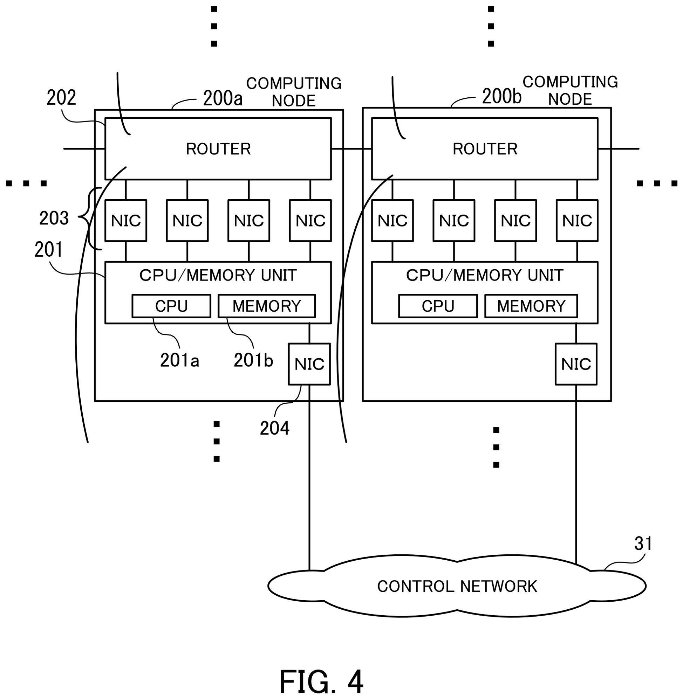

FIG. 4 illustrates an example of a hardware configuration of a computing node. The computing node 200a includes a CPU/memory unit 201 and a router 202. The CPU/memory unit 201 and the router 202 are connected by a plurality of communication interfaces (NICs) 203. An NIC 204 is also connected to the CPU/memory unit 201 for connection to the control network 31.

The CPU/memory unit 201 includes a CPU 201a having a plurality of cores, and a memory 201b. In the CPU/memory unit 201, a process (execution unit of processing) is generated for each core. In the case where a process running on a core of the CPU/memory unit 201 synchronizes with processes in other computing nodes, the process communicates with those other computing nodes 200b, ... via the router 202.

The router 202 communicates with a computing node adjacent to each other in a three-dimensional direction. The router 202 transmits data to be transmitted from the CPU/memory unit 201 to another computing node, to its adjacent computing node in a direction corresponding to the position of the computing node in the interconnect 32. When the router 202 receives data for a process in the CPU/memory unit 201 from its adjacent computing node, the router 202 transmits the data to the CPU/memory unit 201. Further, in the case where the router 202 receives, from its adjacent computing node, data addressed to another computing node, the router 202 transmits the data to its adjacent computing node in a direction corresponding to the position of the transmission destination computing node in the interconnect 32.

The other computing nodes 200b, ... each have the same hardware configuration as the computing node 200a. The computing nodes 200a, 200b, ... with the hardware as illustrated in FIG. 4 are connected in a mesh/torus topology in a three-dimensional direction to form the three-dimensional mesh/torus interconnect 32.

The processing functions of the second embodiment may be implemented by the control node 100 and the computing nodes 200a, 200b, ... configured with the hardware described above. The information processing apparatus 10 described in the first embodiment may also be implemented with hardware similar to that of the control node 100 or the computing node 200a.

In the computing system 30, the stable structure of a molecule constituting a substance is computed by performing structure optimization using DFT. Here, the reason why the use of DFT in the structure optimization increases the computational complexity will be described.

In the structure optimization, the atomic positions are updated and the DFT calculation is performed with the updated atomic arrangement, iteratively, until the forces acting on all atoms arranged in the space or in the coordinate system converge. The force refers to the magnitude of force (e.g., Coulomb force) acting on each atom. If the forces on all atoms are sufficiently small, the structure of the molecule at that time is stable. Such a convergence criterion is expressed by the following formula.

$$\text{Convergence criterion: } |F_i| \leq |F| \qquad (1)$$

Here, i is the identification number of an atom included in the molecule. $|F_i|$ is the magnitude of the force on the i-th atom. $|F|$ is the allowable value of the force. The total energy of the molecule when all the atoms satisfy the convergence criterion is taken as the total energy in the stable structure.

If any one of the following conditions is satisfied, the total energy transitions into minute fluctuations.

The first condition is that there exists an atom that has a small influence on the total energy (i.e., an atom with low energy) and the atom remains unconverged. For example, an atom with low energy refers to an atom that has only electrons in orbitals (electron shells) close to the atomic nucleus. In addition, even an atom having an electron in an outer electron shell becomes a low-energy atom in a stable structure if there exists another atom forming a pair sharing that electron.

The second condition is the occurrence of vibrations of an atom with low energy (i.e., periodic displacements of the atom due to the force acting on the atom).

If there exists an atom that satisfies either of these conditions, the total energy may transition to minute fluctuations. If the total energy transitions to minute fluctuations, atoms whose forces do not satisfy the above convergence criteria remain indefinitely. As a result, the number of iterations of the DFT calculation in the structure optimization increases, which leads to a prolonged computation time.

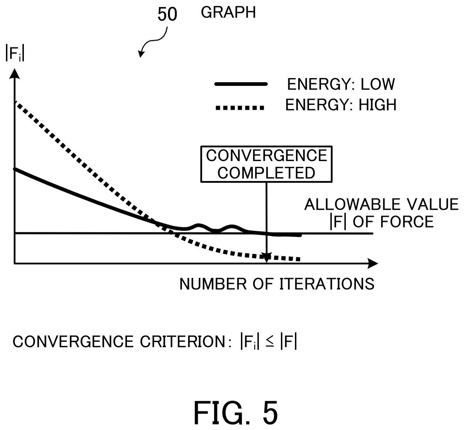

FIG. 5 illustrates the relationship between the magnitude of energy of an atom and the ease of convergence of force. In a graph 50 illustrated in FIG. 5, the horizontal axis represents the number of DFT iterations, and the vertical axis represents the force $|F_i|$ acting on an atom. The solid curve in the graph 50 represents changes in the force with an increase in the number of DFT iterations, for an atom with low energy (for example, an atom with the minimum energy). The dashed curve in the graph 50 represents changes in the force with an increase in the number of DFT iterations, for an atom with high energy (for example, an atom with the maximum energy).

The graph 50 illustrates an example in which only the convergence condition for force is used as the convergence condition. For an atom with high energy, the force falls below the allowable value at an early stage as the DFT iterations progress. On the other hand, for an atom with low energy, the force smoothly decreases to a certain degree as the number of DFT iterations increases; however, even if the number of DFT iterations increases thereafter, the force slowly decreases while repeatedly increasing and decreasing. For this reason, the number of DFT iterations needed until the force satisfies the convergence criterion is greater for atoms with low energy than for atoms with high energy.

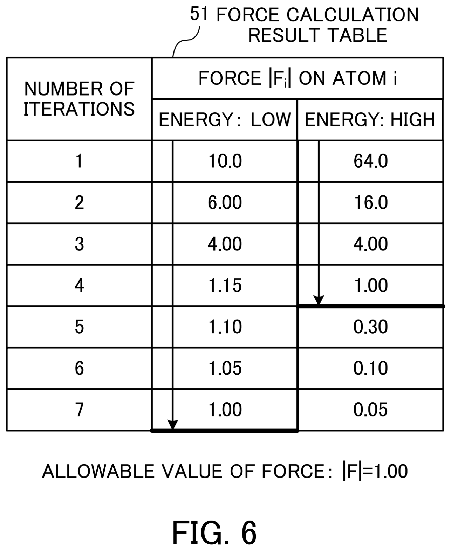

FIG. 6 illustrates an example of the number of DFT iterations needed until the convergence condition of force is satisfied. A force calculation result table 51 represents the calculation result of a force acting on an atom obtained in each DFT iteration, for an atom with low energy and an atom with high energy. The allowable value of force is "1.00".

As seen in the force calculation result table 51, the force on the atom with high energy is "1.00", which is less than or equal to the allowable value, at the fourth DFT calculation. On the other hand, the force on the atom with low energy is "1.00" which is less than or equal to the allowable value, at the seventh DFT calculation.

The convergence condition in the structure optimization is that the forces on all atoms satisfy the convergence criterion. In the case where the calculation results of force as seen in the force calculation result table 51 are obtained, the DFT calculation needs to be repeated seven times until the structure optimization process converges.

As described above, since the ease of the force convergence greatly differs for each atom, the number of DFT iterations needed until the convergence is completed for all atoms is large.

Considering the above, the computing system 30 according to the second embodiment determines whether the structure optimization has converged, by a logical OR of a convergence condition (first convergence condition) related to force and a convergence condition (second convergence condition) related to the degree of a change in total energy (energy change rate (RCE)).

The first convergence condition is that the forces on all atoms satisfy the convergence criterion (i.e., the force become less than or equal to the allowable value "$|F|$" of force).

The second convergence condition is that the energy change rate satisfies the convergence criterion (i.e., the energy change rate becomes less than or equal to the allowable value "RCE_TH" of the energy change rate).

The energy change rate is a value representing the degree of a change between the previous total energy and the current total energy. The energy change rate is given by, for example, the following equation.

```
Energy Change Rate (RCE)

    = |(current value - previous value) ÷ previous value|    (2)
```

The current value is the total energy value obtained from the result of the most recent DFT. The previous value is the total energy value obtained from the result of the DFT immediately before the most recent DFT. Instead of the previous value, a representative value (for example, an average value) of the total energy obtained from the results of past several DFTs may be used. For example, in the case where the most recent DFT is the n-th DFT, the past several DFTs may be the m-th ($m \leq n$) to (n-1)-th DFTs.

If either of the two convergence conditions is satisfied, the computing system 30 determines that the calculation result of the structure optimization has converged.

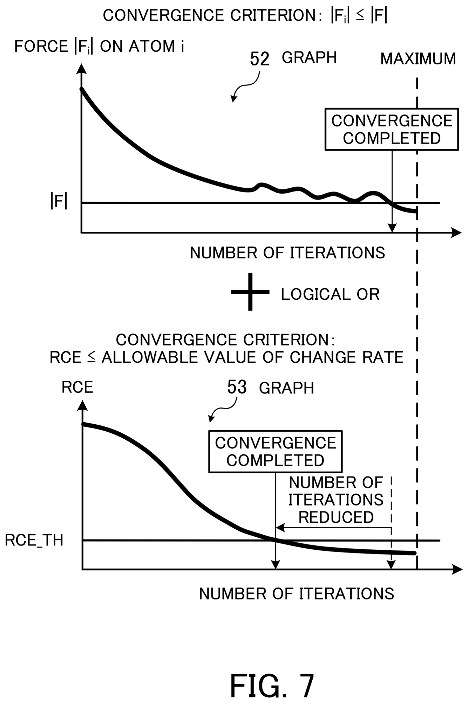

FIG. 7 illustrates an example of convergence determination in the structure optimization using a logical OR of two convergence conditions. A graph 52 represents the determination result of the first convergence condition. The horizontal axis of the graph 52 represents the number of DFT iterations, and the vertical axis represents the force "$|F_i|$" on an atom. In the graph 52, the curve represents changes in the force with an increase in the number of DFT iterations, with respect to an atom with low energy. The horizontal line in the graph 52 represents the allowable value "$|F|$" of the force.

A graph 53 represents the determination result of the second convergence condition. The horizontal axis of the graph 53 represents the number of DFT iterations, and the vertical axis represents the energy change rate "RCE". In the graph 53, the curve represents the changes in the total energy of the molecule with an increase in the number of DFT iterations. The horizontal line in the graph 53 represents the allowable value "RCE_TH" of the energy change rate.

As seen from the comparison between the graph 52 and the graph 53, the energy change rate satisfying the convergence criterion is earlier than the force on the atom satisfying the convergence criterion. That is, by determining the convergence of the structure optimization using not only the first convergence condition but also the second convergence condition, the number of DFT iterations needed until convergence is reduced. As a result, the calculation time for the structure optimization is also reduced.

Even if the updating of the positions of the atoms and the DFT calculation are repeated until the first convergence condition is satisfied in the case illustrated in FIG. 7, the structure of the substance (the arrangement of the atoms) does not change much from that at the time when the second convergence condition is satisfied.

The arrangement of atoms obtained through the structure optimization may be a local optimum solution. A local optimum solution is a solution in which the total energy is minimized in a local space, and is not a solution (global optimum solution) in which the total energy is minimized in the entire search space.

In the actual structure optimization, the user confirms whether the arrangement of atoms when the convergence condition is satisfied is a local optimum solution. For example, in the case where the total energy is higher than that in a similar structure optimization performed in the past, it is considered to be a local optimum solution. In the case of a local optimum solution, the user changes the initial positions of the atoms, for example, and causes the computing system 30 to perform the structure optimization again.

A local optimum solution may be obtained as described above. Therefore, even if the structure optimization is continued until the first convergence condition is satisfied after the second convergence condition is satisfied, this may merely improve the accuracy of the positions of the atoms in the local optimum solution. Therefore, in the case where the second convergence condition is satisfied, the structure optimization process is terminated, and the process of checking whether the results is a local optimum solution is performed. By doing so, recalculation of the structure optimization, which is performed when a local optimum solution is obtained, may be performed at an early stage, thereby improving the efficiency of searching for a stable structure of the substance.

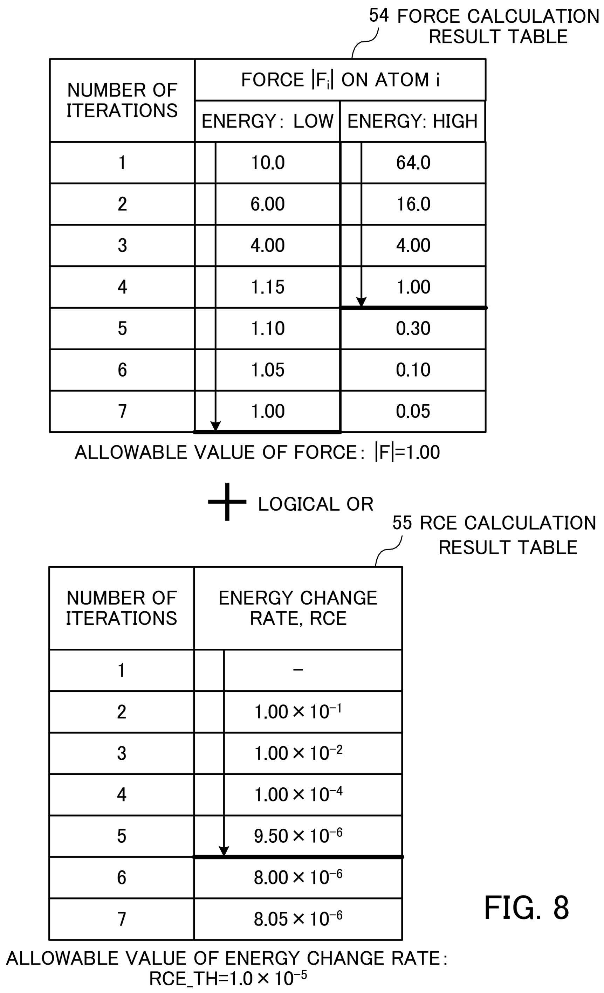

FIG. 8 illustrates an example of the number of DFT iterations needed until two convergence conditions are satisfied. A force calculation result table 54 represents the calculation result of a force acting on an atom obtained in each DFT iteration, for an atom with low energy and an atom with high energy. Here, it is assumed that the allowable value of force is "1.00".

As seen in the force calculation result table 54, the force on the atom with high energy is "1.00", which is less than or equal to the allowable value, at the fourth DFT calculation. On the other hand, the force on the atom with low energy is "1.00", which is less than or equal to the allowable value, at the seventh DFT calculation.

An RCE calculation result table 55 represents the calculation result of the energy change rate "RCE" obtained in each DFT iteration. Here, it is assumed that the allowable value "RCE_TH" of the total energy is $1.0 \times 10^{-5}$.

As seen in the RCE calculation result table 55, the energy change rate obtained by DFT decreases as the number of iterations increases, and the energy change rate becomes less than or equal to the allowable value at the fifth DFT calculation.

Referring to the example of FIG. 8, the first convergence condition related to force is satisfied at the seventh DFT calculation, whereas the second convergence condition related to the energy change rate is satisfied at the fifth DFT calculation. By taking the logical OR of the first convergence condition and the second convergence condition, it is determined that the calculation of the structure optimization has converged at the fifth DFT calculation. That is, the computational complexity for determining the stable structure of the molecule is reduced.

In this connection, even if an atom remains whose force exceeds the allowable value, it is considered that, if the energy change rate is less than or equal to the allowable value, the total energy does not decrease significantly even if the DFT calculation is further repeated. Therefore, even if the second convergence condition is added as the convergence condition in the structure optimization, the finally obtained total energy becomes a value close to the minimum value. That is, the stable structure of the molecule may be identified with sufficiently high accuracy.

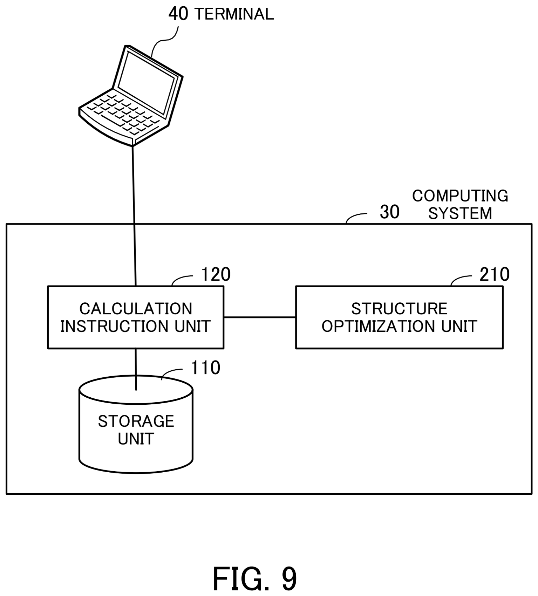

FIG. 9 is a block diagram illustrating an example of functions implemented by the computing system. The computing system 30 includes a storage unit 110, a calculation instruction unit 120, and a structure optimization unit 210.

The storage unit 110 and the calculation instruction unit 120 are functions provided by the control node 100, for example. The structure optimization unit 210 is a function provided by, for example, the computing nodes 200a, 200b, ....

The storage unit 110 stores information (such as contained atoms) on a substance to be calculated.

When the calculation instruction unit 120 receives an analysis instruction for a stable structure of a predetermined substance, from the terminal 40, the calculation instruction unit 120 instructs the structure optimization unit 210 to perform a structure optimization process for the substance.

The structure optimization unit 210 calculates the positions of atoms of a molecule that provide a stable structure of the molecule, using DFT. For example, the structure optimization unit 210 arranges the atoms contained in the molecule in a space to be analyzed. The structure optimization unit 210 then calculates the electron density in the space for the arrangement of the atoms, using DFT. In the calculation of the electron density, the structure optimization unit 210 iteratively performs an SCF calculation until the total energy in the current atomic arrangement is minimized. When the minimum value of the total energy in the current arrangement of the atoms is obtained, the structure optimization unit 210 moves the atoms so as to further reduce the energy. The structure optimization unit 210 performs such DFT calculation and updating of the positions of the atoms iteratively. Thus, the arrangement of the atoms that minimizes the total energy is obtained. The arrangement of the atoms with the minimum total energy is the stable structure of the molecule.

The function of each element illustrated in FIG. 9 may be implemented by causing a computer to execute a program module corresponding to that element, for example. For example, the control node 100 executes a program corresponding to the calculation instruction unit 120 to implement the calculation instruction unit 120. The calculation instruction unit 120 implemented in the control node 100 instructs one or more of the plurality of computing nodes 200a, 200b, ... to execute a job corresponding to the structure optimization unit 210. The instructed computing node implements the structure optimization unit 210 by executing a program corresponding to the specified job.

Next, a procedure for the structure optimization process will be described.

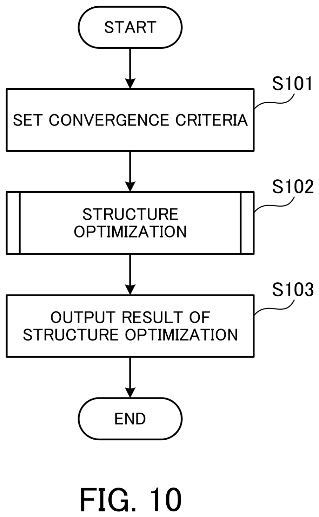

FIG. 10 is a flowchart illustrating an example procedure for the structure optimization process. Hereinafter, the process illustrated in FIG. 10 will be described in order of step numbers.

[Step S101] The calculation instruction unit 120 sets a convergence criterion to be applied to the structure optimization. For example, the user inputs an allowable value "|F|" of force and an allowable value "RCE_TH" of an energy change rate to the terminal 40. The terminal 40 transmits the input allowable value "|F|" of the force and the allowable value "RCE_TH" of the energy change rate to the control node 100. The calculation instruction unit 120 sets the received allowable value "|F|" of the force and the allowable value "RCE_TH" of the energy change rate as parameters indicating convergence criteria, in the job of the structure optimization process.

[Step S102] The calculation instruction unit 120 instructs the structure optimization unit 210 to execute a structure optimization job. Then, the structure optimization unit 210 performs the structure optimization process.

[Step S103] The calculation instruction unit 120 outputs the calculation result of the structure optimization. For example, the calculation instruction unit 120 transmits an analysis result including the coordinates indicating the arrangement of the atoms in the stable structure, the total energy in the stable structure, and others, to the terminal 40. The terminal 40 displays the analysis result on, for example, a monitor.

Next, a procedure for the structure optimization process will be described in detail.

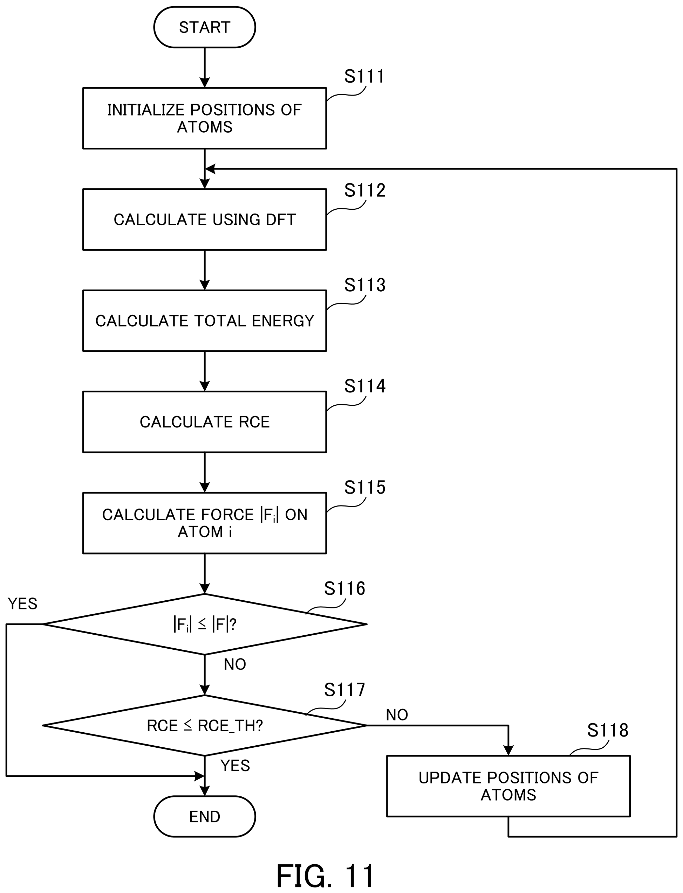

FIG. 11 is a flowchart illustrating an example procedure for the structure optimization process. Hereinafter, the process illustrated in FIG. 11 will be described in order of step numbers.

[Step S111] The structure optimization unit 210 initializes atomic positions. For example, the structure optimization unit 210 sets the coordinates indicating the positions of the atoms included in a molecule to predetermined initial values.

[Step S112] The structure optimization unit 210 calculates the electron density within the space to be analyzed, with respect to the current atomic arrangement using DFT. For example, the SCF method is used to calculate the electron density.

[Step S113] The structure optimization unit 210 calculates the total energy of the molecule, based on the electron density for each coordinate in the space.

[Step S114] The structure optimization unit 210 calculates the energy change rate "RCE" in the second and subsequent DFT calculations.

[Step S115] The structure optimization unit 210 calculates the force "$|F_i|$" on each atom. The force "$|F_i|$" on each atom may be calculated based on the sum of interaction potentials with all other atoms. For example, the structure optimization unit 210 calculates a potential gradient at the position of each atom. The calculated gradient represents the direction and strength of the force acting on the atom.

[Step S116] The structure optimization unit 210 determines for all the atoms whether the force "$|F_i|$" on each atom is less than or equal to the allowable value "|F|" ($|F_i| \leq |F|$). If "$|F_i| \leq |F|$" is satisfied for all the atoms, the structure optimization unit 210 terminates the structure optimization process. If "$|F_i| \leq |F|$" is not satisfied for at least one atom, the structure optimization unit 210 advances the process to step S117.

[Step S117] The structure optimization unit 210 determines whether the energy change rate "RCE" is less than or equal to the allowable value "RCE_TH" of the energy change rate (RCE ≤ RCE_TH). If "RCE ≤ RCE_TH" is satisfied, the structure optimization unit 210 terminates the structure optimization process. If "RCE ≤ RCE_TH" is not satisfied, the structure optimization unit 210 advances the process to step S118. Note that the structure optimization unit 210 may execute step S116 and step S117 in reverse order.

[Step S118] The structure optimization unit 210 updates the positions of the atoms so that the total energy decreases. For example, the structure optimization unit 210 moves each atom in the direction opposite to the force acting on that atom by an amount corresponding to the magnitude of the force. The structure optimization unit 210 is also able to determine the movement direction and the movement amount for each atom using the quasi-Newton method or the conjugate gradient method. Thereafter, the structure optimization unit 210 advances the process to step S112.

In this way, when either the first convergence condition related to the force or the second convergence condition related to the energy change rate is satisfied, the structure optimization is determined to have converged, and the structure optimization process is terminated.

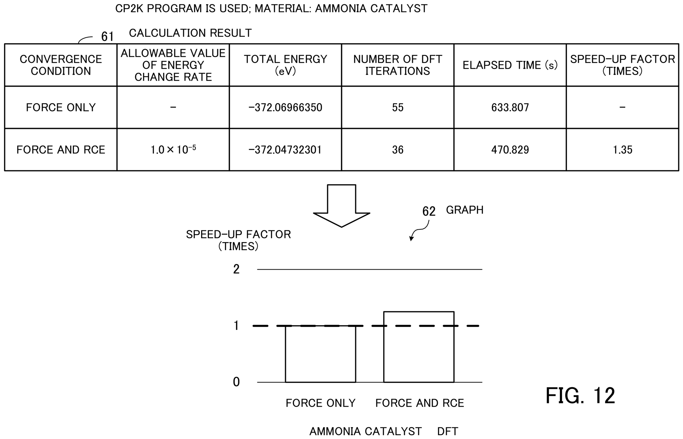

FIG. 12 illustrates an example of speeding up the structure optimization calculation by additionally using the convergence condition related to the energy change rate. FIG. 12 illustrates a calculation result 61 obtained when a stable structure of an ammonia catalyst is obtained by DFT calculation using the CP2K program. As the convergence condition in the structure optimization, two patterns are applied: a case where only the first convergence condition related to force is applied, and a case where the logical OR of the first convergence condition related to force and the second convergence condition related to the energy change rate is applied. The allowable value of the energy change rate is "$1.0 \times 10^{-5}$".

In the case where only the first convergence condition is applied as the convergence determination condition, the total energy is "-372.06966350" (eV), the number of DFT iterations is "55", and the elapsed time until convergence is "633.807" (s). In the case where the first convergence condition and the second convergence condition are applied as the convergence determination condition, the total energy is "-372.04732301" (eV), the number of DFT iterations is "36", the elapsed time until convergence is "470.829" (s), and a speed-up factor is "1.35".

A graph 62 represents a speed-up factor "1" in the case where only the first convergence condition related to force is applied, and a speed-up factor "1.35" in the case where the logical OR of the first convergence condition related to force and the second convergence condition related to the energy change rate is applied.

As described above, the calculation speed is increased by using the logical OR of the first convergence condition related to force and the second convergence condition related to the energy change rate as the convergence condition in the structure optimization. Moreover, as seen in the calculation result 61, there is no significant difference in the obtained total energy. That is, the calculation time is shortened without reducing the calculation accuracy.

When the logical OR of the first convergence condition and the second convergence condition is applied, the total energy at the time when the structure optimization converges is higher by approximately "0.2" (eV) than that in the case where only the first convergence condition is applied. The suitability of a substance as an ammonia catalyst is evaluated based on the adsorption energy, and the quality of the adsorption energy is generally compared in units of "0.5" (eV). Therefore, an error of approximately "0.2" (eV) in the total energy of the structure obtained by the structure optimization is an acceptable error in the search for the ammonia catalyst.

By employing the technology described in the second embodiment, for example, it becomes possible to efficiently implement a wide-area search technology service for new materials in which HPC and artificial intelligence (AI) are integrated.

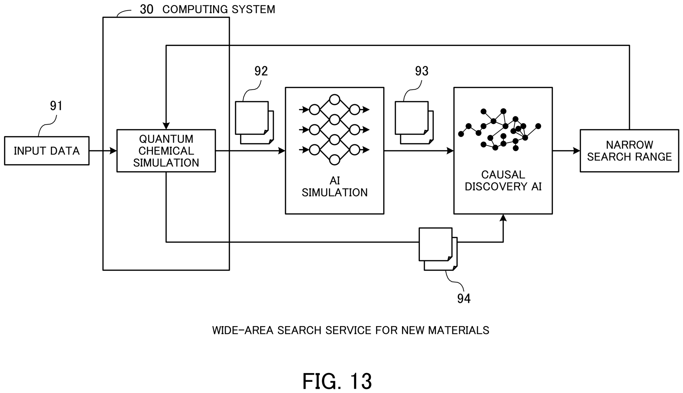

FIG. 13 illustrates an example of a wide-area search technology service. For example, the computing system 30 acquires input data 91 indicating initial conditions related to a substance, such as a material to be searched, from a user. The computing system 30 executes a quantum chemical simulation at high speed based on the input data 91. As a result, high-quality data 92 and 94 relating to the structure of the substance is easily generated.

The data 92 generated by the computing system 30 may be analyzed with respect to characteristics and others by AI simulation using a technique such as a graph neural network (GNN), for example. By using the data 93 generated by the AI simulation and the data 94 generated by the computing system 30, it is possible to analyze causal relationships from combinations of a large number of material candidates, humans are not able to analyze, and physical properties of the materials by using, for example, causal discovery AI. As a result, new discovery and insights may be obtained.

The analysis results of the causal relationships may be used for narrowing the search range, for example, by being reflected in the search conditions of the quantum chemical simulation. By appropriately narrowing the search range, for example, a substance having characteristics desired by the user may be found at an early stage.

[Other Embodiments]

In the second embodiment, an example in which the disclosed technology is applied to a wide-area search service for new materials using AI has been described. However, the disclosed technology is also applicable to other information processing services.

In the second embodiment, the DFT calculation is performed using the HPC. However, In the case where the number of atoms in a substance to be analyzed is small, the structure optimization described in the second embodiment may be performed by a computer other than the HPC.

Although the embodiments have been illustrated, the configuration of each unit described in the embodiments may be replaced with another configuration having the same function. In addition, any other components or processes may be added. Furthermore, any two or more configurations (features) of the above-described embodiments may be combined.

Reference Signs List

1 Substance information
2 Position information
3 First convergence condition
4 Second convergence condition
10 Information processing apparatus
11 Storage unit
12 Processing unit

**Claims**

1. A quantum chemical computation program that causes a computer to perform a process comprising:

    initially setting positions of a plurality of atoms constituting a substance in position information indicating the positions of the plurality of atoms, and iteratively updating the positions of the plurality of atoms in a direction in which a state of the substance becomes stabilized;
    calculating, in response to the positions of the plurality of atoms being initially set or updated, a total energy of the substance based on the position information;
    calculating, in response to the positions of the plurality of atoms being initially set or updated, a magnitude of a force acting on each of the plurality of atoms based on the position information;
    calculating, in response to the positions of the plurality of atoms being updated, an energy change rate indicating a degree of a change in the total energy of the substance before and after the updating;
    determining whether at least one of a first convergence condition related to the magnitude of the force and a second convergence condition related to the energy change rate is satisfied; and
    terminating, upon determining that at least one of the first convergence condition and the second convergence condition is satisfied, the updating of the positions of the plurality of atoms, and acquiring the position information at termination.

2. The quantum chemical computation program according to claim 1, wherein the determining whether at least one of the first convergence condition and the second convergence condition is satisfied includes determining that the first convergence condition is satisfied, upon determining that the magnitude of the force is less than or equal to a first allowable value for all of the plurality of atoms.

3. The quantum chemical computation program according to claim 1, wherein the determining whether at least one of the first convergence condition and the second convergence condition is satisfied includes determining that the second convergence condition is satisfied, upon determining that the energy change rate is less than or equal to a second allowable value.

4. The quantum chemical computation program according to any one of claims 1 to 3, wherein the calculating of the energy change rate includes calculating a difference between a first total energy of the substance based on the position information after n-th updating (n is a natural number) and a representative value of a second total energy of the substance based on the position information after m-th updating (m is a natural number of n - 1 or less) to (n-1)-th updating, dividing the difference by the representative value of the second total energy, and setting an absolute value of a result of the dividing as the energy change rate.

5. A quantum chemical computation method executed by a computer, the quantum chemical computation method comprising:

initially setting positions of a plurality of atoms constituting a substance in position information indicating the positions of the plurality of atoms, and iteratively updating the positions of the plurality of atoms in a direction in which a state of the substance becomes stabilized;

calculating, in response to the positions of the plurality of atoms being initially set or updated, a total energy of the substance based on the position information;

calculating, in response to the positions of the plurality of atoms being initially set or updated, a magnitude of a force acting on each of the plurality of atoms based on the position information;

calculating, in response to the positions of the plurality of atoms being updated, an energy change rate indicating a degree of a change in the total energy of the substance before and after the updating;

determining whether at least one of a first convergence condition related to the magnitude of the force and a second convergence condition related to the energy change rate is satisfied; and

terminating, upon determining that at least one of the first convergence condition and the second convergence condition is satisfied, the updating of the positions of the plurality of atoms, and acquiring the position information at termination.

6. An information processing apparatus comprising:
a processing unit configured to:

initially set positions of a plurality of atoms constituting a substance in position information indicating the positions of the plurality of atoms, and iteratively update the positions of the plurality of atoms in a direction in which a state of the substance becomes stabilized;

calculate, in response to the positions of the plurality of atoms being initially set or updated, a total energy of the substance based on the position information;

calculate, in response to the positions of the plurality of atoms being initially set or updated, a magnitude of a force acting on each of the plurality of atoms based on the position information;

calculate, in response to the positions of the plurality of atoms being updated, an energy change rate indicating a degree of a change in the total energy of the substance before and after the updating;

determine whether at least one of a first convergence condition related to the magnitude of the force and a second convergence condition related to the energy change rate is satisfied; and

terminate, upon determining that at least one of the first convergence condition and the second convergence condition is satisfied, the updating of the positions of the plurality of atoms, and acquire the position information at termination.

7. The information processing apparatus according to claim 6, wherein, in determining whether at least one of the first convergence condition and the second convergence condition is satisfied, the processing unit determines that the first convergence condition is satisfied, upon determining that the magnitude of the force is less than or equal to a first allowable value for all of the plurality of atoms.

8. The information processing apparatus according to claim 6, wherein, in determining whether at least one of the first convergence condition and the second convergence condition is satisfied, the processing unit determines that the second convergence condition is satisfied, upon determining that the energy change rate is less than or equal to a second allowable value.

9. The information processing apparatus according to any one of claims 6 to 8, wherein, in calculating the energy change rate, the processing unit calculates a difference between a first total energy of the substance based on the position information after n-th updating (n is a natural number) and a representative value of a second total energy of the substance based on the position information after m-th updating (m is a natural number of n - 1 or less) to (n-1)-th updating, divides the difference by the representative value of the second total energy, and sets an absolute value of a result of the dividing as the energy change rate.

INFORMATION
PROCESSING
APPARATUS
10

STORAGE
UNIT
11

SUBSTANCE
INFORMATION
1

PROCESSING
UNIT
12

ITERATIVELY
UPDATE POSITIONS
OF ATOMS

POSITION
INFORMATION
2

| ATOM | POSITION |
|------|----------|
| 1 | $(x1, y1, z1)$ |
| 2 | $(x2, y2, z2)$ |
| ... | ... |

CALCULATE
MAGNITUDES OF
FORCES ACTING ON
ATOMS

CALCULATE TOTAL
ENERGY (DFT)

CALCULATE ENERGY
CHANGE RATE

| NUMBER OF ITERATIONS | FORCE $|F_i|$ ON ATOM $i$ | | |
|---|---|---|---|
| | $|F_1|$ | $|F_2|$ | ... |
| 1 | 10.0 | 64.0 | ... |
| 2 | 6.00 | 16.0 | ... |
| 3 | 4.00 | 4.00 | ... |
| 4 | 1.15 | 1.00 | ... |
| 5 | 1.10 | 0.30 | ... |

| NUMBER OF ITERATIONS | ENERGY CHANGE RATE (RCE) |
|---|---|
| 1 | – |
| 2 | $1.00 \times 10^{-1}$ |
| 3 | $1.00 \times 10^{-2}$ |
| 4 | $1.00 \times 10^{-4}$ |
| 5 | $9.50 \times 10^{-6}$ |

CONVERGENCE DETERMINATION

FIRST CONVERGENCE
CONDITION
$|F_i| \leq |F|$  $(|F|=1.00)$
3

OR

SECOND CONVERGENCE CONDITION
$RCE \leq RCE\_TH$  $(RCE\_TH = 1.0 \times 10^{-5})$
4

CONVERGENCE AT
FIFTH ITERATION

TERMINATION
OF UPDATING

ACQUIRE POSITION INFORMATION

(POSITIONS OF ATOMS SET AT FIFTH
ITERATION)

FIG. 1

FIG. 2

FIG. 3

FIG. 4

CONVERGENCE CRITERION : $|F_i| \leq |F|$

# FIG. 5

51 FORCE CALCULATION
RESULT TABLE

| NUMBER OF ITERATIONS | FORCE $|F_i|$ ON ATOM $i$ | | | |
|---|---|---|---|---|
| | ENERGY: LOW | | ENERGY: HIGH | |
| 1 | | 10.0 | | 64.0 |
| 2 | | 6.00 | | 16.0 |
| 3 | | 4.00 | | 4.00 |
| 4 | | 1.15 | | 1.00 |
| 5 | | 1.10 | | 0.30 |
| 6 | | 1.05 | | 0.10 |
| 7 | | 1.00 | | 0.05 |

ALLOWABLE VALUE OF FORCE: $|F|=1.00$

# FIG. 6

CONVERGENCE CRITERION: $|F_i| \leq |F|$

FORCE $|F_i|$ ON ATOM i

52 GRAPH

MAXIMUM

CONVERGENCE
COMPLETED

$|F|$

NUMBER OF ITERATIONS

╋ LOGICAL OR

CONVERGENCE CRITERION:
RCE ≤ ALLOWABLE VALUE OF CHANGE RATE

RCE

53 GRAPH

CONVERGENCE
COMPLETED

NUMBER OF
ITERATIONS
REDUCED

RCE_TH

NUMBER OF ITERATIONS

FIG. 7

54 FORCE CALCULATION
RESULT TABLE

| NUMBER OF ITERATIONS | FORCE $|F_i|$ ON ATOM i | |
|---|---|---|
| | ENERGY: LOW | ENERGY: HIGH |
| 1 | 10.0 | 64.0 |
| 2 | 6.00 | 16.0 |
| 3 | 4.00 | 4.00 |
| 4 | 1.15 | 1.00 |
| 5 | 1.10 | 0.30 |
| 6 | 1.05 | 0.10 |
| 7 | 1.00 | 0.05 |

ALLOWABLE VALUE OF FORCE: $|F|=1.00$

$+$ LOGICAL OR

55 RCE CALCULATION
RESULT TABLE

| NUMBER OF ITERATIONS | ENERGY CHANGE RATE, RCE |
|---|---|
| 1 | – |
| 2 | $1.00 \times 10^{-1}$ |
| 3 | $1.00 \times 10^{-2}$ |
| 4 | $1.00 \times 10^{-4}$ |
| 5 | $9.50 \times 10^{-6}$ |
| 6 | $8.00 \times 10^{-6}$ |
| 7 | $8.05 \times 10^{-6}$ |

ALLOWABLE VALUE OF ENERGY CHANGE RATE:
$RCE\_TH = 1.0 \times 10^{-5}$

FIG. 8

40 TERMINAL

COMPUTING
SYSTEM
30

120

CALCULATION
INSTRUCTION UNIT

210

STRUCTURE
OPTIMIZATION UNIT

110

STORAGE
UNIT

FIG. 9

START

SET CONVERGENCE CRITERIA — S101

STRUCTURE
OPTIMIZATION — S102

OUTPUT RESULT OF
STRUCTURE OPTIMIZATION — S103

END

# FIG. 10

START

INITIALIZE POSITIONS OF ATOMS — S111

CALCULATE USING DFT — S112

CALCULATE TOTAL ENERGY — S113

CALCULATE RCE — S114

CALCULATE FORCE $|F_i|$ ON ATOM $i$ — S115

$|F_i| \leq |F|$? — S116

YES

NO

RCE $\leq$ RCE_TH? — S117

NO

YES

UPDATE POSITIONS OF ATOMS — S118

END

FIG. 11

CP2K PROGRAM IS USED; MATERIAL: AMMONIA CATALYST

61   CALCULATION RESULT

| CONVERGENCE CONDITION | ALLOWABLE VALUE OF ENERGY CHANGE RATE | TOTAL ENERGY (eV) | NUMBER OF DFT ITERATIONS | ELAPSED TIME (s) | SPEED-UP FACTOR (TIMES) |
|---|---|---|---|---|---|
| FORCE ONLY | – | −372.06966350 | 55 | 633.807 | – |
| FORCE AND RCE | $1.0 \times 10^{-5}$ | −372.04732301 | 36 | 470.829 | 1.35 |

62   GRAPH

SPEED-UP FACTOR (TIMES)

2

1

0

FORCE ONLY   FORCE AND RCE

AMMONIA CATALYST   DFT

FIG. 12

30 COMPUTING SYSTEM

91
INPUT DATA

QUANTUM
CHEMICAL
SIMULATION

92

AI
SIMULATION

93

CAUSAL
DISCOVERY AI

94

NARROW
SEARCH RANGE

WIDE-AREA SEARCH SERVICE FOR NEW MATERIALS

FIG. 13

## INTERNATIONAL SEARCH REPORT

| International application No. |
| --- |
| **PCT/JP2024/010198** |

### A. CLASSIFICATION OF SUBJECT MATTER

*G16C 10/00*(2019.01)i
FI: G16C10/00

According to International Patent Classification (IPC) or to both national classification and IPC

### B. FIELDS SEARCHED

Minimum documentation searched (classification system followed by classification symbols)

G16C10/00-99/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| A | JP 2012-32908 A (TOYOTA JIDOSHA KABUSHIKI KAISHA) 16 February 2012 (2012-02-16) entire text | 1-9 |
| A | WO 2015/132865 A1 (FUJITSU LIMITED) 11 September 2015 (2015-09-11) entire text | 1-9 |
| A | JP 2002-113370 A (TOYOTA JIDOSHA KABUSHIKI KAISHA) 16 April 2002 (2002-04-16) entire text | 1-9 |

☐ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **30 May 2024** | **11 June 2024** |

| Name and mailing address of the ISA/JP | Authorized officer |
| --- | --- |
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (July 2022)

**INTERNATIONAL SEARCH REPORT**
Information on patent family members

International application No.

**PCT/JP2024/010198**

| Patent document cited in search report | Publication date (day/month/year) | Patent family member(s) | Publication date (day/month/year) |
|---|---|---|---|
| JP 2012-32908 A | 16 February 2012 | (Family: none) | |
| WO 2015/132865 A1 | 11 September 2015 | US 2016/0357891 A1 entire text | |
| JP 2002-113370 A | 16 April 2002 | (Family: none) | |

Form PCT/ISA/210 (patent family annex) (July 2022)

**REFERENCES CITED IN THE DESCRIPTION**

**Patent documents cited in the description**

- JP 6187404 A